(19) Europäisches Patentamt
European Patent Office
Office européen des brevets

(11) **EP 2 070 470 B1**

(12) **EUROPEAN PATENT SPECIFICATION**

(45) Date of publication and mention
of the grant of the patent:
**01.12.2010 Bulletin 2010/48**

(51) Int Cl.:
***A61B 5/00*** *(2006.01)*

(21) Application number: **08171057.6**

(22) Date of filing: **09.12.2008**

(54) **Electronic device, arrangement, and method of estimating fluid loss**

Elektronische Vorrichtung, Anordnung und Verfahren zur Beurteilung des Flüssigkeitsverlusts

Dispositif électronique, agencement et procédé pour estimer la perte de fluides

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **14.12.2007 FI 20075910**

(43) Date of publication of application:
**17.06.2009 Bulletin 2009/25**

(73) Proprietor: **Polar Electro Oy**
**90440 Kempele (FI)**

(72) Inventor: **Nissilä, Juuso**
**91100, Ii (FI)**

(74) Representative: **Antila, Harri Jukka Tapani**
**Kolster Oy Ab**
**P.O. Box 148**
**Iso Roobertinkatu 23**
**00121 Helsinki (FI)**

(56) References cited:
**DE-U1-202007 018 496      FR-A- 2 863 474**
**GB-A- 2 411 719            US-A- 6 138 079**
**US-A1- 2007 082 789**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF THE INVENTION

The present invention relates to an electronic device, an arrangement, a method, and a computer-readable distribution medium.

BACKGROUND OF THE INVENTION

Perspiration (also called sweating or sometimes transpiration) is the production and evaporation of a fluid, consisting primarily of water as well as a smaller amount of sodium chloride excreted by the sweat glands in the skin. Sweating is primarily a means of thermoregulation. Evaporation of sweat from the skin surface has a cooling effect due to the latent heat of evaporation of water. Thus, in hot weather, or when an individual's muscles heat up due to exercise, more sweat is produced.

Sweating eliminates waste heat that is formed during muscular work. Without eliminating this waste heat, the inner temperature of an individual's body would rise to a level that may threaten one's health and life very quickly. However, since sweating effectively eliminates water from the organs, it has to be replaced somehow. Intense sweating for a long period of time leads to dehydration, i.e. to a condition in which the body contains an insufficient volume of water for normal functioning. Dehydration also weakens the ability of the body to remove waste heat by sweating. Sweating may also lead to disturbances in the ion balance of the body that may, in turn, lead to serious disturbances of the central nervous system (nausea, faintness, cramps, arrhythmia, convulsions). Indirectly, dehydration may cause hypertermia because of the decreased ability to sweat, for instance. The symptoms of hypertermia include, for example, decreased feeling of thirstiness, irritability, confusion, aggression, euphoria, disturbances of consciousness, blackout, and death. Heart-originated symptoms include, for example, disturbances of conduction, ST (*tachycardia sinualis*) changes and T-wave inversions.

Traditionally, the amount of dehydration caused by sweating has been modelled as a function of inner temperature, surface temperature of the skin and environment. The phenomenon of dehydration is difficult to model and, thus its modelling is challenging. One of the known modelling attempts dates back to 1970's (Nadel *et al*. 1973). The known methods aim to control the heat flux starting from the increased inner temperature of the body. The heat flux aims to turn outwards towards a lower thermal potential. The known models take at least one of the following parameters into account: the size of the individual (the distance from the core to the surface, the area of the skin evaporating heat), thermal gradient (wet bulb globe temperature, WBGT), the capacitive and conductive properties affecting the conduction of heat in each medium (emissive power of skin, heat convection capacity of blood circulation of skin, heat accumulation ability of tissues, permeability of vapour, convection and radiation of clothing).

Thermal dissipation is a very dynamic phenomenon and it is transformed as the load increases. Heat dissipation in the skin is weighted in different ways in different situations. For example, skin that turns glossy because of sweating evaporates and radiates differently than dry skin. Further, as the properties of clothing change, a clothing index should be known; the dampness of cloth changes its properties of heat conduction, permeability and radiation. One of the problems related to the known solutions is that clothing and skin are given static values. Further, the known solutions are oriented such that heat distribution has to be known first in order to determine conduction/convection and radiation, and sweating is only responsible for the rest. Accordingly, more effective techniques for determining the amount of dehydration caused by sweating are needed.

US 6138079 discloses a device for calculating fluid loss.

BRIEF DESCRIPTION OF THE INVENTION

An object of the present invention is to provide an improved method, an electronic device, an arrangement, and a computer-readable distribution medium. The objects of the invention are achieved by an electronic device, an arrangement, and a method that are characterized by what is stated in the independent claims.

According to an aspect of the invention, there is provided an electronic device comprising: a processing unit configured to receive skin temperature data generated by a measuring unit, to receive performance data from a measuring unit, and to determine a theoretical fluid loss value on the basis of the received performance data. The electronic device further comprises: a processing unit configured to determine a relation between a predetermined perspiration threshold and a skin temperature value deduced from the received skin temperature data; and to determine a real fluid loss value on the basis of the theoretical fluid loss value and the determined relation between the predetermined perspiration threshold and the skin temperature value.

According to another aspect of the invention, there is provided an arrangement comprising: a measuring unit configured to measure skin temperature data; a measuring unit configured to measure performance data; a receiving unit configured to receive the measured skin temperature data and to receive the measured performance data; and a calculator configured to determine a theoretical fluid loss value on the basis of the received performance data. The arrangement further comprises: a calculator configured to determine a relation between a predetermined perspiration threshold and a skin temperature value deduced from the received skin temperature data; and a calculator configured to determine a real fluid loss value on the basis of the theoretical fluid loss value and the determined relation between the predetermined perspiration threshold and the skin temperature value.

According to another aspect of the invention, there is provided a method of estimating fluid loss, the method comprising: receiving skin temperature data, receiving performance data, and determining a theoretical fluid loss value on the basis of the received performance data. The method further comprises: determining a relation between a predetermined perspiration threshold and a skin temperature value deduced from the received skin temperature data; and determining a real fluid loss value on the basis of the theoretical fluid loss value and the determined relation between the predetermined perspiration threshold and the skin temperature value.

According to another aspect of the invention, there is provided a computer program comprising program instructions which, when loaded into an electronic device, cause the electronic device to perform the process comprising: receiving skin temperature data, receiving performance data, and determining a theoretical fluid loss value on the basis of the received performance data. The process further comprises: determining a relation between a predetermined perspiration threshold and a skin temperature value deduced from the received skin temperature data; and determining a real fluid loss value on the basis of the theoretical fluid loss value and the determined relation between the predetermined perspiration threshold and the skin temperature value.

The invention is based on approaching fluid loss estimation via a thermodynamic reduction process. Skin temperature values are used to estimate more accurate estimates on actual values of fluid loss, i.e. perspiration.

The electronic device and method of the invention provide several advantages. Estimating more accurate values for fluid loss/perspiration is possible. Different instructions based on real fluid loss may, thus, be generated.

BRIEF DESCRIPTION OF THE DRAWINGS

In the following the invention will be described in greater detail with reference to the embodiments and the accompanying drawings, in which

Figure 1 shows an example of the structure of an arrangement according to an embodiment;
Figure 2 shows an example of the structure of an electronic device according to an embodiment;
Figure 3 shows an example of an arrangement according to an embodiment;
Figure 4 shows another example of an arrangement according to an embodiment;
Figure 5 shows an example of the relation between fluid loss, the maximum amount of perspiration, the maximum fluid consuming ability, and an optimal fluid consuming amount; and
Figure 6 shows an example of a method of estimating fluid loss.

DETAILED DESCRIPTION OF THE INVENTION

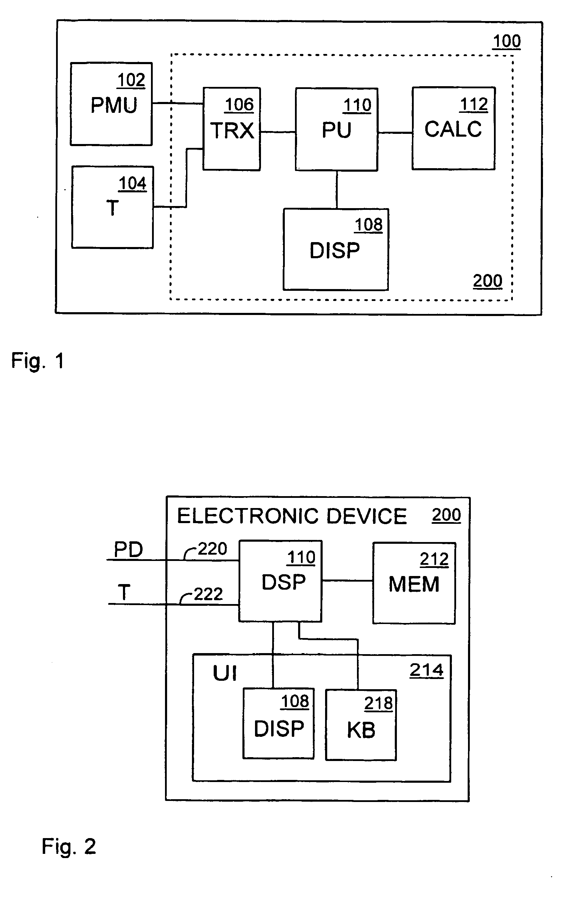

With reference to Figure 1, we now examine an example of an arrangement to which embodiments of the invention can be applied. The embodiments are, however, not restricted to this arrangement described only by way of example, but a person skilled in the art can apply the instructions to other arrangements containing corresponding characteristics.

The arrangement 100 of Figure 1 comprises a performance measuring unit 102, a skin temperature measuring unit 104, a receiving unit 106, a control unit 110, a calculation unit 112, and a display unit 108. The different elements of this arrangement 100 may be separate devices that can communicate with one or more other elements of the arrangement. In the example of Figure 1, the receiving unit 106, the control unit 110, the calculation unit 112, and the display unit 108 may be physically included in a single electronic device 200, and the performance measuring unit 102 and the skin temperature measuring unit 104 may form their own entities that communicate with the electronic device 200 with wired or wireless connections. However, the performance measuring unit 106 and/or the skin temperature measuring unit 104 may also be parts of the electronic device 200.

The skin temperature measuring unit 104 is configured to measure skin temperature data of a user of the device. The measured skin temperature data may comprise skin temperature values measured from different parts of the user's body. The measured skin temperature data is transmitted to the receiving unit 106. The skin temperature data may be used to deduce a weighted mean value of the measured skin temperature values.

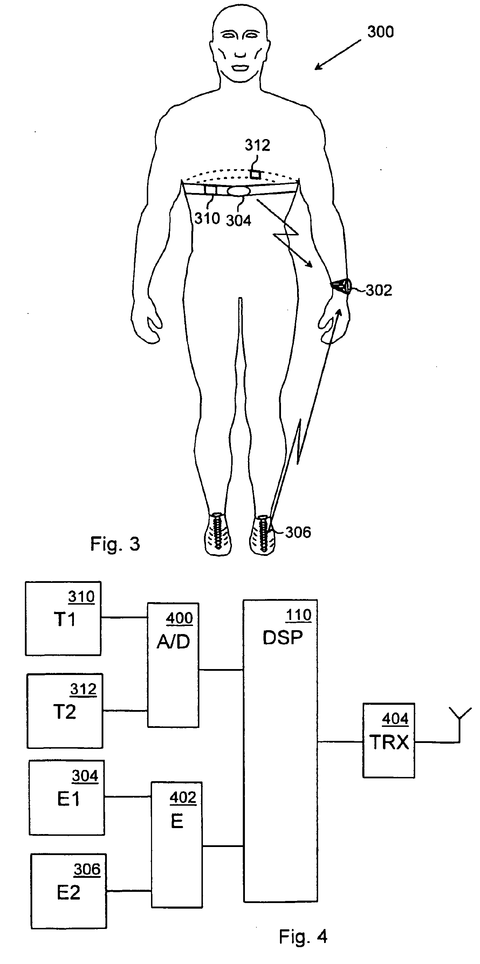

In an embodiment, the skin temperature measuring unit 104 may comprise or be part of a wrist device that may be the wrist device 302 of a performance monitor shown in Figure 3. A performance monitor may comprise not only the wrist device 302, but also one or more auxiliary devices 304, 306, 310, 312 such as a motion sensor 306 fastened to a limb of the user 300 of the device and/or a heart rate transmitter 304 indicating electric pulses induced by the heart.

The example of Figure 3 shows two skin temperature measuring devices 310, 312 fastened to a flexible belt-like structure. In an embodiment, the skin temperature values are measured from different parts of the body, here, from the front part and from the back part of the upper torso area. The two skin temperature measuring devices 310, 312 of this example are arranged such that temperature measuring may be performed from the opposite sides of the body. This arrangement of the temperature measuring devices 310, 312 is beneficial since the measurement points provide temperature values that are close to those obtained from optimised multi-point measurement.

If more than two skin temperature measuring devices are used, they can be arranged at predetermined distances from each other. For example, it is possible to use any given number of sensors, 6, 7 or 15 sensors, for example, arranged to measure skin temperature from many different parts of the body. In an embodiment, the skin temperature is measured from anatomically different parts of the body because the skin temperature varies depending on the body part, e.g. the skin temperature in the front part of the torso may differ from the skin temperature in the back. The measured skin temperature data is used to calculate a weighted mean value of the measured skin temperature values.

In an embodiment, a first skin temperature measuring device 310 is fastened to a non-flexible part of the belt-like structure, whereas a second skin temperature measuring device 312 is fastened to a flexible part of the belt-like structure. This way the distance between the skin temperature measuring devices may be easily controlled by adjusting the belt between the skin temperature measuring devices. The skin temperature measuring devices 310, 312 may also be fastened to any other structures and e.g. to clothing, such as a shirt, a bra, and suspenders. The skin temperature measuring devices 310, 312 may also be attached against the user's skin with glue, if necessary. The auxiliary devices 304, 306, 310, 312 of Figure 3 may communicate over wired or wireless connections with the wrist device 302. In an embodiment, the motion sensor 306 comprises an acceleration sensor that measures the acceleration related to the movement of the user 300. The acceleration sensor transforms the acceleration caused by a movement or gravity into an electric signal.

The temperature measuring devices 310, 312 may be based on prior art temperature gauges, such as thermocouples or thermal resistors.

The performance measuring unit 102 is configured to measure performance data of a user of the device. The measured performance data may comprise performance parameters such as: heart rate (cardiac output), heart rate variation, any threshold value, velocity, reciprocal of velocity, pedalling power, cadence, pace frequency, activity parameter, pulse, power level, step length, mechanical measurement, experimental value, any physiological parameter, or any ratio thereof, or any combination thereof. Any suitable methods and elements, such as pulse detectors and acceleration sensors, can be used to measure these performance parameters. In an embodiment, the performance-measuring unit 102 may comprise or be part of a wrist device that may be the wrist device 302 of a performance monitor shown in Figure 3.

The receiving unit 106 is configured to receive the measured performance data from the measuring units 102, 104, and the calculator 112 is configured to determine a theoretical fluid loss value on the basis of the received performance data.

The skin temperature measuring unit 104 and the performance-measuring unit 102 may communicate with the receiving unit 106 over wireless or wired connections. It is possible that the electronic device 200 is a personal computer, a PDA (Personal Digital Assistant) device, a handheld computer, or any portable device, and the data from the skin temperature measuring unit 104 and the performance data are loaded to the device 200 for further processing. It is also possible that the data from the skin temperature measuring unit 104 and the performance measuring unit 102 is directly and continuously deliverer to the receiving unit 106 while the data is being measured in the skin temperature measuring unit 104 and the performance measuring unit 102.

In an embodiment, the skin temperature measuring unit 104 is configured to determine and store skin temperature data and the performance-measuring unit 102 is configured to measure and store performance data continuously for a certain period of time, after which the stored skin temperature data and the performance data are transferred to the receiving unit 106. It is also possible that the determined skin temperature data and/or the performance data is not stored in the skin temperature measuring unit 104 or in the performance measuring unit 102 but is continuously communicated via a communication connection to the receiving unit 106.

The calculator 112 is configured to determine a relation between a predetermined perspiration threshold and a skin temperature value deduced from the received skin temperature data. The calculator 112 is further configured to determine a real fluid loss value on the basis of the theoretical fluid loss value and the determined relation between the skin temperature value and the predetermined perspiration threshold.

In an embodiment of the invention, the controller 110 is configured to generate a performance instruction based on the determined real fluid loss value.

The controller 110 comprises a digital signal processor and executes a computer process according to encoded instructions stored in a memory. The processing unit 110 may be implemented by using analog circuits, ASIC circuits (Application Specific Integrated Circuit), a digital processor, a memory, and computer software. The controller 110 may constitute part of the computer of a wrist device 302, for example.

The display unit 108 that may contain LCD (Liquid Crystal Display) components, for instance, may indicate the generated performance instructions to the user 300.

Figure 2 shows another example of the structure of an electronic device 200 according to an embodiment. The electronic device 200 typically comprises a controller 110, a memory unit 212, and user interface parts 214. The electronic device 200 may be, for example, a personal computer, a wrist device 302, a device carried on a bicycle, an exercise equipment at the gym, and/or a military application for monitoring military training, for example.

The controller 110 receives skin temperature data 222 from a measuring unit and performance data 220 from a measuring unit. A calculation of an average mean value of the skin temperature values included in the skin temperature data can be executed in the controller 110 or in any another processing device, for example in the skin temperature measuring unit 104 or in the wrist device 302.

The controller 110 controls the functions of the electronic device 200, and it may execute computer processes according to encoded instructions stored in the memory unit 212. The calculator 112 of Figure 1 may be part of the controller 110.

The user interface 214 typically comprises a display unit 108 and a display controller. The user interface 214 may further comprise a keypad 218 allowing the user to input commands in the electronic device 200. The display unit 108 is configured to indicate generated performance instructions.

In an embodiment, the electronic device 200 may comprise a pulse counter, in which case the electronic device 200 receives a signal 222 transmitted from the performance-measuring unit 102. The performance measuring unit 102 may, for example, be a belt-like structure installed on the user's chest and comprise means for performing an electrocardiogram measurement (ECG) and for transmitting ECG information to the electronic device 200.

In an embodiment, signal 222 includes heart rate information, such as heart rate, heart pulse intervals, and/or heart rate variation in digitally or analogically coded form.

In an embodiment, the controller 110 is configured to determine a relation between a predetermined perspiration threshold and a skin temperature value deduced from the received skin temperature data; to determine a real fluid loss value on the basis of the theoretical fluid loss value and the determined relation between the skin temperature value and the predetermined perspiration threshold; and to generate a performance instruction based on the determined real fluid loss value. Thus, exercisers may be given specific instructions that are based on real fluid loss values.

In an embodiment, the determined real fluid loss value is smaller than the theoretical fluid loss value if the skin temperature value is less than the predetermined perspiration threshold, the determined real fluid loss value is approximately the theoretical fluid loss value if the skin temperature equals the predetermined perspiration threshold, and determined real fluid loss value is greater than the theoretical fluid loss value if the skin temperature is greater than the predetermined perspiration threshold.

In an embodiment, the controller 110 is configured to determine a skin temperature coefficient factor on the basis of a relation between the skin temperature value and the predetermined perspiration threshold. The skin temperature coefficient factor characterises the difference between the theoretical and real amount of sweating, i.e. the difference between perfectly evaporated water capable to dissipate all generated extra heat and the real amount of water required to cool the body.

In an embodiment, the controller 110 is configured to determine the real fluid loss value by using the determined skin temperature coefficient factor and the theoretical fluid loss value. In an example, a product of the determined skin temperature coefficient factor and the theoretical fluid loss value may be used to determine the real fluid loss value.

In an embodiment, the value of the skin temperature coefficient factor increases as a function of the skin temperature.

In an embodiment, the skin temperature value is a weighted mean value of the received skin temperature data, the skin temperature data including two or more skin temperature values measured from different parts of the body.

In an embodiment, the controller 110 is further configured to estimate an amount of fluids to be consumed on the basis of the determined real fluid loss value, and the generated performance instruction includes an instruction of consuming the estimated amount of fluids. In an embodiment, the instruction for consuming fluids or beverages may contain information on the correct amount of fluids and their concentration, or the amount of dissolved ingredients (e.g. amount of sodium chloride or other salts, or other osmolality increasing components such as sugars, longer-chain carbohydrates, amino acids or proteins). Fluid consumption instruction may be based on information of estimated fluid loss, exercise duration and intensity. Hence, forecasting optimal rehydration during differing loads from short and intense to long-lasting low-intense (even repeated several days hiking or parching) is enabled.

In an embodiment, the controller 110 is further configured to generate a performance instruction to dress or undress depending on the determined relation between the skin temperature value and the predetermined perspiration threshold. Dressing instructions may be given when a threshold value falls below a predetermined perspiration threshold or a value derived from that, and undressing instructions may be given when a threshold value exceeds a predetermined perspiration threshold or a value derived from that. Dressing or undressing instructions may comprise advice given in terms of an insulative layer number, clothing index, clothing mass, clothing ventilation (such as using openings, adjustable properties of intelligent clothing), or may be configured to change with the skin temperature, i.e. no further clothing addition/removal is instructed when a predetermined threshold value (e.g. slightly under perspiration threshold value when thermoneutral performance conditions are desirable and possible, like during a longer walk outdoors) is reached.

In an embodiment, the controller 110 may take a planned load into account when generating a performance instruction to dress or undress. The planned load is the load derived from an individual training plan of a user. For example, getting one's clothes wet is not harmful during a short-term exercise whereas during a wintry ski tour or hike it may be detrimental.

Figure 4 shows another example of an arrangement according to an embodiment. The arrangement comprises a first skin temperature measuring device 310, a second skin temperature measuring device 312, an analogue-to-digital converter 400, a first performance measuring device 304, a second performance measuring device 306, a pre-processing device 402, a digital signal processor 110, and a transmitter 404.

The skin temperature values measured by the skin temperature measuring devices 310, 312 are provided via the analogue-to-digital converter 400 to the digital signal processor 110. The performance data measured by the performance measuring devices 304, 306 are provided via the pre-processing device 402 to the digital signal processor 110. The pre-processing device 402 may process primary performance data, such as heart rate data, acceleration data, and/or vibration data. The processing may comprise transforming primary motion data into secondary motion data, for instance transforming acceleration data related to a user-generated movement into motion pulse data. The processing may also comprise filtering primary and/or secondary performance data.

The digital signal processor 110 determines a theoretical fluid loss value on the basis of the received performance data, determines a relation between a predetermined perspiration threshold and a skin temperature value deduced from the received skin temperature data, determines a real fluid loss value on the basis of the theoretical fluid loss value and the determined relation between the skin temperature value and the predetermined perspiration threshold.

In an embodiment, the digital signal processor 110 generates a performance instruction based on the determined real fluid loss value. The generated performance instruction may be transmitted by the transmitter 404 to another device, such as a computer. For example, a trainer/coach may receive performance instructions relating to individual players in his sports team by using a receiving device, such as a computer. Thus, the trainer may give specified instructions on the amounts of fluid the players should consume during an exercise or a game. In an embodiment, the generated performance instruction may be based on the estimated duration of an exercise in addition to the determined real fluid loss value. In an embodiment, the performance instruction may include instructions on the level of intensity to be followed during an exercise, i.e. instructions may be given in order for the user to avoid heat shock, excessive water loss and fatigue.

In an embodiment, the arrangement of Figure 4 is located in the pulse transmitter 304 of Figure 3. The fluid loss information may be communicated to a wrist device and/or a base station of a multi-user central processing unit, such as a team base station. In such a case, a coach may monitor the fluid status of the team.

Let us now study some theoretical basis of determining real fluid loss. In an embodiment, a theoretical fluid loss value may be estimated by any known means and methods on the basis of the received performance data. In an embodiment, the theoretical fluid loss value determination starts by determining a biological work power $W_B$. The biological work power is a measure of energy consumption due to physical work. The biological work power may be determined in many different ways on the basis of the received performance data. In an embodiment, the biological work power $W_B$ may be determined by using equation 1:

$$W_B = W_O{}^2 = 5 \cdot W_{pd} = W_{running} \qquad (1)$$

where $W_O$ is a measure of oxygen consumption, $W_{pd}$ is a measure of work power by pedalling power, and $W_{running}$ is a measure of work power by running which may be determined on the basis of running speed and transforming it into biological work using known or developed equations. The biological work power may be determined experimentally.

Next, a measure of power of lost heat $W_W$ may be determined, for example by using equation 2:

$$W_W = a \cdot W_B \qquad (2),$$

where $a$ is a coefficient related to how much of the total work is wasted. In an embodiment, it may be assumed that 70 to 80% of total work is wasted, thus the coefficient $a$ can be between 0.7-0.8.

A theoretical evaporation power $W_{evap}$ equals the power of lost heat $W_W$. The theoretical fluid loss value $M_{H2Ot}$ [g/h/W] may be determined from the theoretical evaporation power $W_{evap}$, for example, by using equation 3:

$$M_{H2Ot} = W_{evap} \cdot 1.486 g/h \tag{3}.$$

The value of 1.486 g/h of equation 3 is determined based on heat of evaporation of water, and it can be found in literature.

In an embodiment, the skin temperature value may be determined by taking a mean value of the received skin temperature data. For example, in the case where two skin temperature values are measured, equation 4 can be used for determining the skin temperature value $T_{sk}$:

$$T_{sk} = \frac{T_{front} + T_{back}}{2} \tag{4},$$

where $T_{front}$ is skin temperature measured from the front of the torso, and $T_{back}$ is a skin temperature value measured from the back part of the torso.

In an embodiment, a skin temperature coefficient factor $K_{sk}$ is determined on the basis of a relation between the skin temperature value and the predetermined perspiration threshold $PT$. The perspiration threshold $PT$ may vary individually, for example between 32 and 36°C. In an embodiment, the perspiration threshold is approximately 34°C. Table 1 illustrates an example of how the skin temperature coefficient factor may be determined:

Table 1. Relationship between skin temperature $T_{sk}$, skin temperature coefficient factor $K_{sk}$, theoretical fluid loss $M_{H2Ot}$, and real fluid loss $M_{H2Or}$ values.

| $T_{sk}$ | Explanation | $K_{sk}$ | Result |
|---|---|---|---|
| $T_{sk} < PT$ | Heat removal need is smaller than evaporation capacity. Conduction, convection and radiation are in main role. | $K_{sk} < 1$ | $M_{H2Or} < M_{H2Ot}$ Fluid loss amount is small and it can be easily compensated. Very extended load is possible. |
| $T_{sk} = PT$ | Evaporation is working effectively enough: fluid exits by evaporating thus binding heat, and skin stays dry and skin temperature stays reasonable. | $K_{sk} \sim 1$ | $M_{H2Or} \sim M_{H2Ot}$ Perspiration amount is significant (0.8 to 1.2 BW%/h). Upper limit for the duration and power of physical load is set. |
| $T_{sk} > PT$ | Evaporation is underpowered in relation to heat removal need. Heat starts to accumulate to the skin. | $K_{sk} > 1$ | $M_{H2Or} > M_{H2Ot}$ Skin heats up and gets wet. Shining of skin hinders radiation of heat and evaporation. |
| $T_{sk} \gg PT$ | Evaporation is significantly underpowered in relation to heat removal need. Heat accumulates powerfully to the skin as heat removal is being prevented. | $K_{sk} \gg 1$ | $M_{H2Or} \gg M_{H2Ot}$ Skin heats up and gets sub stantially wet. Clothing is wet, thus preventing vapour permeability. Inner temperature increases substantially fast. Extreme fluid loss and disturbance of ion balance if long duration. |

It can be seen from table 1 that the value of the skin temperature coefficient factor $K_{sk}$ varies depending on the relation between the skin temperature value and the predetermined perspiration threshold $PT$. In an embodiment, the skin temperature coefficient factor $K_{sk}$ varies usually between the values of 0.7 and 1.6 but it can be greater than that depending on the situation, for instance it can be greater than 2.

As seen from table 1, when heat begins to accumulate to human tissue, a layer of liquid water is formed over the skin, thus preventing all the excess moist from evaporating. This is one of the reasons why the theoretical fluid loss should be redefined by taking also the skin temperature coefficient factor into account. Clothing, work power and outside air temperature also affect evaporation ability.

In an embodiment, the real fluid loss $M_{H2Or}$ value may be calculated by using equation 5:

$$M_{H2Or} = K_{sk} \cdot M_{H2Ot} \qquad (5).$$

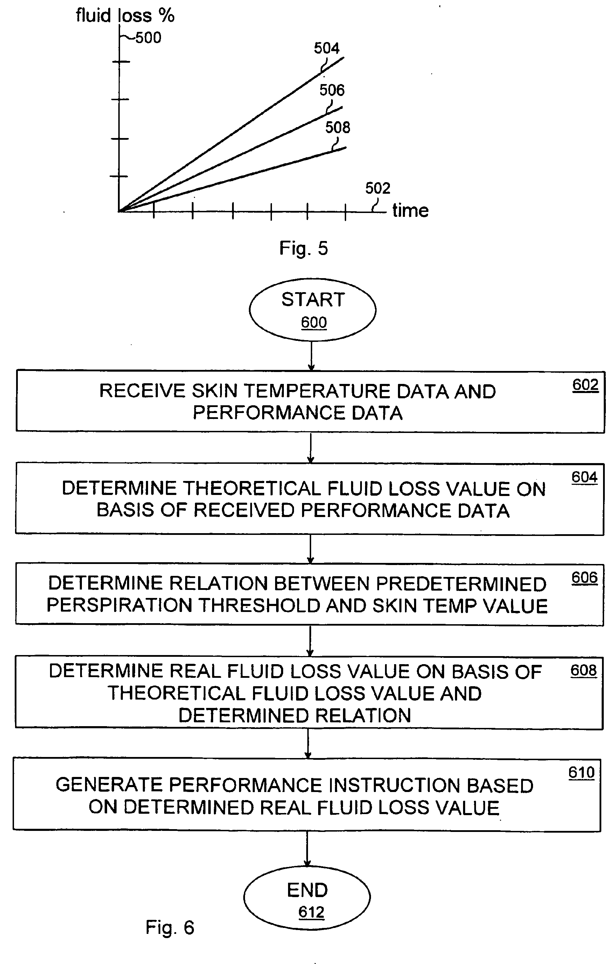

Based on the determined real fluid loss value, it is thus possible to generate different performance instructions in order to stabilize the current physical imbalance of an exerciser. In an embodiment, based on the determined real fluid loss value, an amount of fluid can be determined that the user should consume in a specific situation. Figure 5 shows an example of the rel a-tion between fluid loss 500, the maximum amount of perspiration 504, the maximum fluid consuming ability 508, and an optimal fluid consuming amount 506.

In Figure 5, x-axis 502 represents time and y-axis 500 represents fluid loss percentage during an exercise. It can be seen that perspiration is a linear phenomena in the first approximation. It can also be seen that during maximum perspiration, an exerciser is not able to consume enough fluids to compensate the fluid loss caused by the perspiration. The curve of optimal fluid consuming amount 506 is between the curves of the maximum amount of perspiration 504 and the maximum fluid consuming ability 508. In an embodiment, this can be taken into account by determining the amount of fluids one should consume during the exercise and the amount of fluids one should consume some time after the exercise. Thus, the generated performance instruction may comprise different instructions for the exercise event and after the exercise.

In an embodiment, many different parameters may be taken into account when generating the performance instructions based on the determined real fluid loss. For example, the maximum heart rate value with the maximum fluid consuming amount can be determined, and the performance instruction may, for example, comprise a safety zone for a specific heart rate area that the user should follow in order to exercise without risk. The intensity of the exercise may also be taken into account such that the shorter and intensive the exercise is, the less amount of fluid is to be consumed during the exercise. The rest of the fluid loss may then be compensated during a recovery period. When determining the performance instructions, the amount of urine secretion during the exercise may also be taken into account.

In an embodiment, information on different thresholds may be indicated to the user. These thresholds may indicate to the user health effects/risks he/she may encounter with a specific fluid loss value. For example, such indications may comprise information whether the user is in a balanced state, in a state threatening one's performance, in a state threatening one's health, or in a state threatening one's life. For example, an approximately 2% fluid loss of body weight may threaten one's performance, a greater than 2% fluid loss of body weight may threaten one's health, and an over 4% fluid loss is already life threatening. Over-consuming fluids may also be fatal, and thus, it is also advantageous to indicate the correct amount of fluids one should be drinking. For example, it may be estimated that over-consuming fluids in an amount of 6% of one's body weight may result in edema and even death.

The determined real fluid loss information may also be used as a parameter when modelling exhaustion, determining the amount of clothing one should wear, and/or as a part of a larger concept, such as an intelligent drinking bottle that communicates with a wrist device, for example. As part of team software, the real fluid loss information can directly be communicated to a trainer or a coach responsible for delivering fluids to the athletes during an exercise.

Figure 6 shows an example of a method of estimating fluid loss. The method starts in 600.

In 602, skin temperature data and performance data are received.

In 604, a theoretical fluid loss value is determined on the basis of the received performance data.

In 606, a relation between a predetermined perspiration threshold and a skin temperature value deduced from the received skin temperature data is determined.

In 608, a real fluid loss value is determined on the basis of the theoretical fluid loss value and the determined relation between the skin temperature value and the predetermined perspiration threshold.

In 610, according to an embodiment, a performance instruction is generated based on the determined real fluid loss value.

The method ends in 612.

The embodiments of the invention may be implemented in an electronic device comprising a processing unit. The processing unit may be configured to perform at least some of the steps described in connection with the flowchart of Figure 6 and in connection with Figures 1 to 5. The embodiments may be implemented as a computer program comprising instructions for executing a computer process for estimating fluid loss. A computer process according to an embodiment comprises: receiving skin temperature data, receiving performance data, and determining a theoretical fluid loss value on the basis of the received performance data. The computer process: further comprises determining a relation between a predetermined perspiration threshold and a skin temperature value deduced from the received skin temperature data; determining a real fluid loss value on the basis of the theoretical fluid loss value and the determined relation between the skin temperature value and the predetermined perspiration threshold; and generating a performance instruction based on the determined real fluid loss value.

The computer program may be stored on a computer program distribution medium readable by a computer or a processor. The computer-readable program medium may be, for example but not limited to, an electric, magnetic, optical, infrared, or semiconductor system, device, or transmission medium. The computer program medium may include at least one of the following media: a computer readable medium, a program storage medium, a record medium, a computer readable memory, a random access memory, an erasable programmable read-only memory, a computer readable software distribution package, a computer readable signal, a computer readable telecommunications signal, computer readable printed matter, and a computer readable compressed software package.

It will be obvious to a person skilled in the art that, as the technology advances, the inventive concept can be implemented in various ways. The invention and its embodiments are not limited to the examples described above but may vary within the scope of the claims.

## Claims

1. An electronic device, comprising:

   a processing unit (110) configured to receive skin temperature data generated by a measuring unit, to receive performance data from a second measuring unit, and to determine a theoretical fluid loss value on the basis of the received performance data, wherein the electronic device further comprises the processing unit (110) configured:

   to determine a relation between a predetermined perspiration threshold and a skin temperature value deduced from the received skin temperature data; and
   to determine a real fluid loss value on the basis of the theoretical fluid loss value and the determined relation between the predetermined perspiration threshold and the skin temperature value.

2. The electronic device of claim 1, **characterized in that** the processing unit (110) is further configured to generate a performance instruction based on the determined real fluid loss value.

3. The electronic device of claim 1. **characterized in that** the determined real fluid loss value is smaller than the theoretical fluid loss value if the skin temperature value is less than the predetermined perspiration threshold, approximately the theoretical fluid loss value if the skin temperature equals the predetermined perspiration threshold, and greater than the theoretical fluid loss value if the skin temperature is greater than the predetermined perspiration threshold.

4. The electronic device of claim 1. **characterized in that** the electronic device further comprises a calculator configured to determine a skin temperature coefficient factor on the basis of a relation between the skin temperature value and the predetermined perspiration threshold.

5. The electronic device of claim 4, **characterized in that** the calculator is configured to determine the real fluid loss value by using the determined skin temperature coefficient factor and the theoretical fluid loss value.

6. The electronic device of claim 4, **characterized in that** the value of the skin temperature coefficient factor increases as a function of the skin temperature.

7. The electronic device of claim 1, **characterized in that** the skin temperature value is a weighted mean value of the received skin temperature data, the skin temperature data including two or more skin temperature values measured from different parts of the body.

8. The electronic device of claim 2, **characterized in that** the processing unit is further configured to estimate an amount of fluids to be consumed on the basis of the determined real fluid loss value, and the generated performance instruction includes an instruction for consuming the estimated amount of fluids.

9. The electronic device of claim 1, **characterized in that** the processing unit is further configured to generate a performance instruction to dress or undress depending on the determined relation between the skin temperature value and the predetermined perspiration threshold.

**10.** A method of estimating fluid loss, the method comprising:

receiving skin temperature data,
receiving performance data, and
determining a theoretical fluid loss value on the basis of the received performance data,
determining a relation between a predetermined perspiration threshold and a skin temperature value deduced from the received skin temperature data; and
determining a real fluid loss value on the basis of the theoretical fluid loss value and the determined relation between the predetermined perspiration threshold and the skin temperature value.

**11.** The method of claim 10, **characterized by** the method further comprising: generating a performance instruction based on the determined real fluid loss value.

**12.** The method of claim 10, **characterized in that** the determined real fluid loss value is smaller than the theoretical fluid loss value if the skin temperature value is less than the predetermined perspiration threshold, approximately the theoretical fluid loss value if the skin temperature equals the predetermined perspiration threshold, and greater than the theoretical fluid loss value if the skin temperature is greater than the predetermined perspiration threshold.

**13.** The method of claim 10, **characterized by** further comprising determining a skin temperature coefficient factor on the basis of a relation between the skin temperature value and the predetermined perspiration threshold.

**14.** The method of claim 10, **characterized by** further comprising estimating an amount of fluids to be consumed on the basis of the determined real fluid loss value, and the generated performance instruction includes an instruction of consuming the estimated amount of fluids.

**15.** The method of claim 10, **characterized by** further comprising generating a performance instruction to dress or undress depending on the determined relation between the skin temperature value and the predetermined perspiration threshold.

**16.** A computer program comprising program instructions which, when loaded into an electronic device, cause the electronic device to perform the process of any preceding claim 10 to 15.

**Patentansprüche**

**1.** Elektronische Vorrichtung mit:

einer Verarbeitungseinheit (110), die konfiguriert ist, von einer Messeinheit erzeugte Hauttemperaturdaten zu empfangen, Leistungsdaten von einer zweiten Messeinheit zu empfangen und einen theoretischen Flüssigkeitsverlustwert aufgrund der empfangenen Leistungsdaten zu bestimmen, wobei die elektronische Vorrichtung ferner die Verarbeitungseinheit (110) umfasst, die konfiguriert ist,
ein Verhältnis zwischen einem vorbestimmten Perspirationsgrenzwert und einem von den empfangenen Hauttemperaturdaten abgeleiteten Hauttemperaturwert zu bestimmen; und
einen realen Flüssigkeitsverlustwert aufgrund des theoretischen Flüssigkeitsverlustwerts und des bestimmten Verhältnisses zwischen dem vorbestimmten Perspirationsgrenzwert und dem Hauttemperaturwert zu bestimmen.

**2.** Elektronische Vorrichtung nach Anspruch 1, **dadurch gekennzeichnet, dass** die Verarbeitungseinheit (110) ferner konfiguriert ist, eine Leistungsanweisung aufgrund des bestimmten realen Flüssigkeitsverlustwerts zu erzeugen.

**3.** Elektronische Vorrichtung nach Anspruch 1, **dadurch gekennzeichnet, dass** der bestimmte reale Flüssigkeitsverlustwert kleiner als der theoretische Flüssigkeitsverlustwert ist, falls der Hauttemperaturwert kleiner als der vorbestimmte Perspirationsgrenzwert ist, ungefähr der theoretische Flüssigkeitsverlustwert ist, falls die Hauttemperatur dem vorbestimmten Perspirationsgrenzwert entspricht, und größer als der theoretische Flüssigkeitsverlustwert ist, falls die Hauttemperatur höher als der vorbestimmte Perspirationsgrenzwert ist.

**4.** Elektronische Vorrichtung nach Anspruch 1, **dadurch gekennzeichnet, dass** die elektronische Vorrichtung ferner einen Rechner umfasst, der konfiguriert ist, einen Hauttemperaturkoeffizientsfaktor aufgrund eines Verhältnisses zwischen dem Hauttemperaturwert und dem vorbestimmten Perspirationsgrenzwert zu bestimmen.

**5.** Elektronische Vorrichtung nach Anspruch 4, **dadurch gekennzeichnet, dass** der Rechner konfiguriert ist, den realen Flüssigkeitsverlustwert anhand des bestimmten Hauttemperaturkoeffizientsfaktors und des theoretischen Flüssigkeitsverlustwerts zu bestimmen.

**6.** Elektronische Vorrichtung nach Anspruch 4, **dadurch gekennzeichnet, dass** der Wert des Hauttemperaturkoeffizientsfaktors als Funktion der Hauttemperatur zunimmt.

**7.** Elektronische Vorrichtung nach Anspruch 1, **dadurch gekennzeichnet, dass** der Hauttemperaturwert ein gewogener Mittelwert der empfangenen Hauttemperaturdaten ist, wobei die Hauttemperaturdaten zwei oder mehr von den verschiedenen Teilen des Körpers gemessene Hauttemperaturwerte aufweisen.

**8.** Elektronische Vorrichtung nach Anspruch 2, **dadurch gekennzeichnet, dass** die Verarbeitungseinheit ferner konfiguriert ist, eine aufzubrauchende Flüssigkeitsmenge aufgrund des bestimmten realen Flüssigkeitsverlustwerts zu schätzen, und die erzeugte Leistungsanweisung eine Anweisung zum Aufbrauchen der geschätzten Flüssigkeitsmenge aufweist.

**9.** Elektronische Vorrichtung nach Anspruch 1, **dadurch gekennzeichnet, dass** die Verarbeitungseinheit ferner konfiguriert ist, eine Leistungsanweisung zum Anziehen oder Ausziehen je nach dem bestimmten Verhältnis zwischen dem Hauttemperaturwert und dem vorbestimmten Perspirationsgrenzwert zu erzeugen.

**10.** Verfahren zur Schätzung von Flüssigkeitsverlust, umfassend:

Empfangen von Hauttemperaturdaten,
Empfangen von Leistungsdaten und
Bestimmen eines theoretischen Flüssigkeitsverlustwerts aufgrund der empfangenen Leistungsdaten,
Bestimmen eines Verhältnisses zwischen einem vorbestimmten Perspirationsgrenzwert und einem von den empfangenen Hauttemperaturdaten abgeleiteten Hauttemperaturwert; und
Bestimmen eines realen Flüssigkeitsverlustwerts aufgrund des theoretischen Flüssigkeitsverlustwerts und des bestimmten Verhältnisses zwischen dem vorbestimmten Perspirationsgrenzwert und dem Hauttemperaturwert.

**11.** Verfahren nach Anspruch 10, **dadurch gekennzeichnet, dass** es ferner umfasst: Erzeugen einer Leistungsanweisung aufgrund des bestimmten realen Flüssigkeitsverlustwerts.

**12.** Verfahren nach Anspruch 10, **dadurch gekennzeichnet, dass** der bestimmte reale Flüssigkeitsverlustwert kleiner als der theoretische Flüssigkeitsverlustwert ist, falls der Hauttemperaturwert kleiner als der vorbestimmte Perspirationsgrenzwert ist, ungefähr der theoretische Flüssigkeitsverlustwert ist, falls die Hauttemperatur dem vorbestimmten Perspirationsgrenzwert entspricht, und größer als der theoretische Flüssigkeitsverlustwert ist, falls die Hauttemperatur höher als der vorbestimmte Perspirationsgrenzwert ist.

**13.** Verfahren nach Anspruch 10, **dadurch gekennzeichnet, dass** es ferner Bestimmen eines Hauttemperaturkoeffizientsfaktors aufgrund eines Verhältnisses zwischen dem Hauttemperaturwert und dem vorbestimmten Perspirationsgrenzwert umfasst.

**14.** Verfahren nach Anspruch 10, **dadurch gekennzeichnet, dass** es ferner Schätzen einer aufzubrauchenden Flüssigkeitsmenge aufgrund des bestimmten realen Flüssigkeitsverlustwerts umfasst und die erzeugte Leistungsanweisung eine Anweisung zum Aufbrauchen der geschätzten Flüssigkeitsmenge aufweist.

**15.** Verfahren nach Anspruch 10, **dadurch gekennzeichnet, dass** es ferner Erzeugen einer Leistungsanweisung zum Anziehen oder Ausziehen je nach dem bestimmten Verhältnis zwischen dem Hauttemperaturwert und dem vorbestimmten Perspirationsgrenzwert umfasst.

**16.** Computerprogramm mit Programmbefehlen, die, wenn sie in eine elektronische Vorrichtung geladen sind, die elektronische Vorrichtung dazu bringen, den Prozess nach einem der vorhergehenden Ansprüche 10 bis 15 durchzuführen.

**Revendications**

1. Dispositif électronique, comprenant :

   une unité de traitement (110) configurée pour recevoir des données de température cutanée générées par une unité de mesure, pour recevoir des données de performance d'une seconde unité de mesure, et pour déterminer une valeur de perte de fluide théorique sur la base des données de performance reçues, le dispositif électronique comprenant en outre l'unité de traitement (110) configurée :

       pour déterminer une relation entre un seuil de transpiration prédéterminé et une valeur de température cutanée déduite des données de température cutanée reçues ; et
       pour déterminer une valeur de perte de fluide réelle sur la base de la valeur de perte de fluide théorique et de la relation déterminée entre le seuil de transpiration prédéterminé et la valeur de température cutanée.

2. Dispositif électronique selon la revendication 1, **caractérisé en ce que** l'unité de traitement (110) est en outre configurée pour générer une instruction de performance sur la base de la valeur de perte de fluide réelle déterminée.

3. Dispositif électronique selon la revendication 1, **caractérisé en ce que** la valeur de perte de fluide réelle déterminée est inférieure à la valeur de perte de fluide théorique si la valeur de température cutanée est inférieure au seuil de transpiration prédéterminé, approche la valeur de perte de fluide théorique si la température cutanée est égale au seuil de transpiration prédéterminé, et est supérieure à la valeur de perte de fluide théorique si la température cutanée est supérieure au seuil de transpiration prédéterminé.

4. Dispositif électronique selon la revendication 1, **caractérisé en ce que** le dispositif électronique comprend en outre un calculateur configuré pour déterminer un facteur coefficient de température cutanée sur la base d'une relation entre la valeur de température cutanée et le seuil de transpiration prédéterminé.

5. Dispositif électronique selon la revendication 4, **caractérisé en ce que** le calculateur est configuré pour déterminer la valeur de perte de fluide réelle en utilisant le facteur coefficient de température cutanée déterminé et la valeur de perte de fluide théorique.

6. Dispositif électronique selon la revendication 4, **caractérisé en ce que** la valeur du facteur coefficient de température cutanée augmente en fonction de la température cutanée.

7. Dispositif électronique selon la revendication 1, **caractérisé en ce que** la valeur de température cutanée est une valeur moyenne pondérée des données de température cutanée reçues, les données de température cutanée comprenant deux ou plusieurs valeurs de température cutanée mesurées à partir de différentes parties du corps.

8. Dispositif électronique selon la revendication 2, **caractérisé en ce que** l'unité de traitement est en outre configurée pour estimer une quantité de fluides devant être consommée sur la base de la valeur de perte de fluide réelle déterminée, et l'instruction de performance générée comprend une instruction indiquant de consommer la quantité de fluides estimée.

9. Dispositif électronique selon la revendication 1, **caractérisé en ce que** l'unité de traitement est en outre configurée pour générer une instruction de performance indiquant de s'habiller ou se déshabiller en fonction de la relation déterminée entre la valeur de température cutanée et la valeur de transpiration prédéterminée.

10. Procédé d'estimation d'une perte de fluide, le procédé consistant à :

    recevoir des données de température cutanée,
    recevoir des données de performance, et
    déterminer une valeur de perte de fluide théorique sur la base des données de performance reçues,
    déterminer une relation entre un seuil de transpiration prédéterminé et une valeur de température cutanée déduite des données de température cutanée reçues ; et
    déterminer une valeur de perte de fluide réelle sur la base de la valeur de perte de fluide théorique et de la relation déterminée entre le seuil de transpiration prédéterminé et la valeur de température cutanée.

11. Procédé selon la revendication 10, **caractérisé en ce que** le procédé consiste en outre à : générer une instruction de performance sur la base de la valeur de perte de fluide réelle déterminée.

12. Procédé selon la revendication 10, **caractérisé en ce que** la valeur de perte de fluide réelle déterminée est inférieure à la valeur de perte de fluide théorique si la valeur de température cutanée est inférieure au seuil de transpiration prédéterminé, approche la valeur de perte de fluide théorique si la température cutanée est égale au seuil de transpiration prédéterminé, et est supérieure à la valeur de perte de fluide théorique si la température cutanée est supérieure au seuil de transpiration prédéterminé.

13. Procédé selon la revendication 10, **caractérisé en ce qu'**il consiste en outre à déterminer un facteur coefficient de température cutanée sur la base d'une relation entre la valeur de température cutanée et le seuil de transpiration prédéterminé.

14. Procédé selon la revendication 10, **caractérisé en ce qu'**il consiste en outre à estimer une quantité de fluides devant être consommée sur la base de la valeur de perte de fluide réelle déterminée, et l'instruction de performance générée comprend une instruction indiquant de consommer la quantité estimée de fluides.

15. Procédé selon la revendication 10, **caractérisé en ce qu'**il consiste en outre à générer une instruction de performance indiquant de s'habiller ou se déshabiller en fonction de la relation déterminée entre la valeur de température cutanée et le seuil de transpiration prédéterminé.

16. Programme informatique comprenant des instructions de programme qui, lorsqu'elles sont chargées dans un dispositif électronique, entraînent l'exécution par le dispositif électronique du procédé selon l'une quelconque des revendications 10 à 15.

Fig. 1

Fig. 2

Fig. 3

Fig. 4

fluid loss %

Fig. 5

START
600

RECEIVE SKIN TEMPERATURE DATA AND
PERFORMANCE DATA          602

DETERMINE THEORETICAL FLUID LOSS VALUE ON
BASIS OF RECEIVED PERFORMANCE DATA          604

DETERMINE RELATION BETWEEN PREDETERMINED
PERSPIRATION THRESHOLD AND SKIN TEMP VALUE          606

DETERMINE REAL FLUID LOSS VALUE ON BASIS OF
THEORETICAL FLUID LOSS VALUE AND
DETERMINED RELATION          608

GENERATE PERFORMANCE INSTRUCTION BASED
ON DETERMINED REAL FLUID LOSS VALUE          610

END
612

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6138079 A